(19) Europäisches Patentamt / European Patent Office / Office européen des brevets

(11) **EP 4 454 672 A1**

(12) **EUROPEAN PATENT APPLICATION**
published in accordance with Art. 153(4) EPC

(43) Date of publication:
**30.10.2024 Bulletin 2024/44**

(21) Application number: **22910483.1**

(22) Date of filing: **15.09.2022**

(51) International Patent Classification (IPC):
*A61L 101/20* (2006.01)     *A61L 2/28* (2006.01)
*C12M 1/00* (2006.01)     *C12M 1/12* (2006.01)

(52) Cooperative Patent Classification (CPC):
**A61L 2/28; C12M 1/00; C12M 1/12; A61L 2101/00**

(86) International application number:
**PCT/JP2022/034638**

(87) International publication number:
**WO 2023/119757 (29.06.2023 Gazette 2023/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.12.2021 JP 2021210950**

(71) Applicant: **Nitta Corporation**
**Osaka-shi, Osaka 556-0022 (JP)**

(72) Inventors:
• **SHIGETA, Makoto**
  **Yamatokooriyama-shi, Nara 639-1085 (JP)**
• **IKEDA, Takuji**
  **Yamatokooriyama-shi, Nara 639-1085 (JP)**

(74) Representative: **Hasegawa, Kan**
**Patentanwaltskanzlei Hasegawa**
**Untere Hauptstraße 56**
**85354 Freising (DE)**

(54) **DECONTAMINATION INTENSITY SENSOR, DECONTAMINATION SYSTEM, DECONTAMINATION INTENSITY DETECTION METHOD, AND DECONTAMINATION INTENSITY DETECTION PROGRAM**

(57)     This decontamination intensity sensor detects the degree of decontamination of microorganisms and/or viruses decontaminated with peracetic acid, the decontamination intensity sensor including an exposed part that is made of a metal that is corroded by the peracetic acid, and the metal is exposed.

EP 4 454 672 A1

**Description**

TECHNICAL FIELD

The present invention relates to a decontamination intensity sensor, a decontamination system, a decontamination intensity detection method, and a decontamination intensity detection program.

BACKGROUND ART

Patent Document 1 discloses a decontamination system configured to decontaminate the inside of a safety cabinet as an example of a decontamination object with peracetic acid. In the decontamination system, whether or not the decontamination has been completed is determined using a biological indicator disposed in the safety cabinet.

PRIOR ART DOCUMENT

PATENT DOCUMENT

Patent Document 1: Japanese Patent No. 6067384

SUMMARY OF THE INVENTION

PROBLEMS TO BE SOLVED BY THE INVENTION

In the decontamination system, bacteria and the like adhering to the biological indicator are cultured in a culture solution, and it is determined whether or not decontamination of the decontamination object has been completed based on a change in color of the culture solution. It takes at least about one week to culture bacteria and the like. Therefore, in the decontamination system, it is not possible to determine whether or not decontamination of the decontamination object has been completed in a short time.

An object of the present invention is to provide a decontamination intensity sensor, a decontamination system, a decontamination intensity detection method, and a decontamination intensity detection program capable of determining whether or not decontamination of a decontamination object has been completed in a short time.

MEANS FOR SOLVING THE PROBLEM

A decontamination intensity sensor according to a first aspect of the present invention is a decontamination intensity sensor that detects a degree of decontamination of at least one of microorganisms and viruses decontaminated with peracetic acid, the decontamination intensity sensor including an exposed portion being made of metal corroded by the peracetic acid, the metal being exposed from the exposed portion.

The inventor(s) of the present application have found that when a decontamination object in which at least one of microorganisms and viruses decontaminated with peracetic acid is present is decontaminated with peracetic acid, corrosion of metal proceeds as decontamination intensity increases, and an electrical resistance value increases. According to the decontamination intensity sensor, the corrosion of the exposed portion proceeds as the decontamination intensity of the decontamination object increases, so that the electrical resistance value of the exposed portion increases. Therefore, for example, when the relationship between the electric resistance value of the exposed portion before the start of decontamination and the electric resistance value of the exposed portion after the start of decontamination satisfies a predetermined condition for determining that decontamination has been completed, it can be determined that the decontamination has been completed. Therefore, it can be determined in a short time that decontamination of the decontamination object has been completed.

A decontamination intensity sensor according to a second aspect of the present invention is the decontamination intensity sensor according to the first aspect, in which the corrosion includes a chemical reaction in which the metal is changed to acetate.

Acetate is soluble in water. According to the decontamination intensity sensor, the corroded portion can be removed by wiping the corroded portion when the exposed portion is corroded with, for example, a nonwoven fabric moistened with pure water.

A decontamination intensity sensor according to a third aspect of the present invention is the decontamination intensity sensor according to the first or second aspect, in which the metal contains at least one of copper and zinc.

According to the decontamination intensity sensor, the corrosion of the exposed portion suitably proceeds due to peracetic acid.

A decontamination intensity sensor according to a fourth aspect of the present invention is the decontamination intensity sensor according to the third aspect, in which the metal is an alloy containing the copper and the zinc.

According to the decontamination sensor, the corrosion of the exposed portion particularly suitably proceeds due to peracetic acid.

A decontamination intensity sensor according to a fifth aspect of the present invention is the decontamination intensity sensor according to any one of the first to fourth aspects, further including: a first sensor unit including the exposed portion; and a second sensor unit made of the metal and masked so as not to corrode the metal.

According to the decontamination intensity sensor, the corrosion of the exposed portion of the first sensor unit proceeds as the decontamination intensity of the decontamination object increases, so that the electric resistance value of the first sensor unit increases. On the other hand, since the second sensor unit is masked, it does not corrode even when the decontamination intensity of the decontamination object becomes high. Therefore, the electric resistance value of the second sensor unit does not substantially change. Therefore, when the relationship between the electric resistance value of the first sensor unit and the electric resistance value of the second sensor unit satisfies the predetermined condition in which it can be determined that decontamination has been completed, it can be determined that the decontamination has been completed. Therefore, it can be determined in a short time that decontamination of the decontamination object has been completed.

A decontamination system according to a sixth aspect of the present invention includes: the decontamination intensity sensor according to any one of the first to fifth aspects; and a decontamination device that ejects the peracetic acid to a decontamination object in which at least one of the microorganisms and the viruses is present.

According to the decontamination system, an effect similar to the effect obtained by the decontamination intensity sensor according to the first aspect can be obtained.

A decontamination system according to a seventh aspect of the present invention is the decontamination system according to the sixth aspect, in which the decontamination device is configured to stop ejection of the peracetic acid when a progress of corrosion of the exposed portion satisfies a predetermined condition under which it can be determined that decontamination has been completed.

According to the decontamination system, it is possible to suppress an increase in the ejection amount of peracetic acid.

A decontamination intensity detection method according to an eighth aspect of the present invention is a decontamination intensity detection method for detecting a degree of decontamination of at least one of microorganisms and viruses decontaminated with peracetic acid using a decontamination intensity sensor, in which the decontamination intensity sensor includes an exposed portion being made of metal corroded by the peracetic acid, the metal being exposed from the exposed portion, and in which the decontamination intensity detection method includes a step of determining whether or not a progress of corrosion of the exposed portion satisfies a predetermined condition under which it can be determined that decontamination has been completed.

According to the decontamination intensity detection method, an effect similar to the effect obtained by the decontamination intensity sensor according to the first aspect can be obtained.

A decontamination intensity detection program according to a ninth aspect of the present invention is a decontamination intensity detection program that detects a degree of decontamination of at least one of microorganisms and viruses decontaminated with peracetic acid using a decontamination intensity sensor, in which the decontamination intensity sensor includes an exposed portion being made of metal corroded by the peracetic acid, the metal being exposed from the exposed portion, and in which the decontamination intensity detection program causes a computer to execute a step of determining whether or not a progress of corrosion of the exposed portion satisfies a predetermined condition under which it can be determined that decontamination has been completed.

According to the decontamination intensity detection program, an effect similar to the effect obtained by the decontamination intensity sensor according to the first aspect can be obtained.

ADVANTAGES OF THE INVENTION

According to the decontamination intensity sensor, the decontamination system, the decontamination intensity detection method, and the decontamination intensity detection program according to the present invention, it is possible to determine whether or not decontamination of the decontamination object has been completed in a short time.

BRIEF DESCRIPTION OF THE DRAWINGS

Fig. 1 is a diagram showing a schematic configuration of a decontamination system according to an embodiment.
Fig. 2 is a diagram showing a schematic configuration of an ejection device of Fig. 1.
Fig. 3 is a diagram showing a schematic configuration of a decontamination intensity sensor of Fig. 1.
Fig. 4 is a flowchart showing an example of a method for calculating a reference ratio RAX.
Fig. 5 is a flowchart showing an example of a decontamination procedure.
Fig. 6 is a diagram showing a schematic configuration of a decontamination system used in a first test.
Fig. 7 is a table showing test results of Test Examples 1 to 7 of the first test.
Fig. 8 is a graph showing test results of Test Example 6 of the first test.
Fig. 9 is a table showing test results of Test Examples 8 and 9 of the first test.
Fig. 10 is a diagram showing a schematic configuration of a decontamination system used in a second test.
Fig. 11 is a table showing test results of Test Example 10 of the second test.
Fig. 12 is a table showing specifications of metal samples used in the third test.
Fig. 13 is a table showing results of a third test.

EMBODIMENTS OF THE INVENTION

Hereinafter, a decontamination system according to an embodiment of the present invention will be described with reference to the drawings. Note that, in the present embodiment, decontamination includes at least one of causing damage to microorganisms and viruses floating in the space or attached to the surface of any object using a drug or the like, reducing the number of surviving microorganisms and viruses, killing the microorganisms and viruses, and detoxifying the microorganisms and viruses.

< 1. Configuration of Decontamination System>

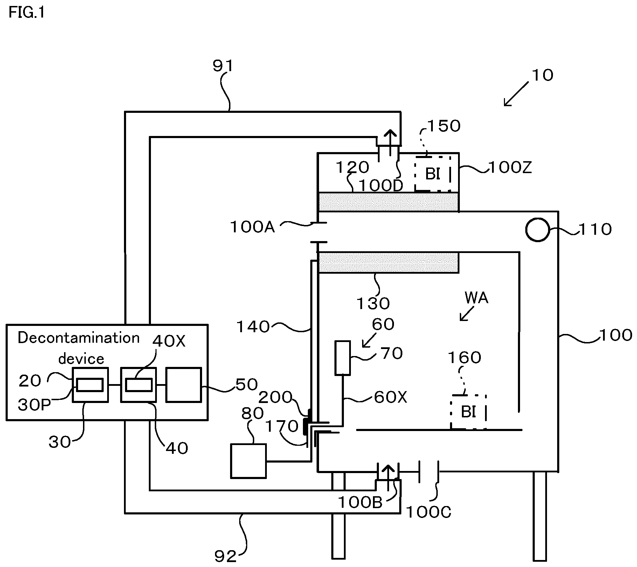

Fig. 1 is a diagram showing a schematic configuration of a decontamination system 10 of the present embodiment. The decontamination system 10 decontaminates a decontamination object 100 using peracetic acid. The decontamination object 100 is, for example, a safety cabinet, an incubator device, an isolator device, a sterile box, an incubator, a centrifuge, a storage, an air conditioning device, a clean bench, a clean room, a duct, or the like. The decontamination object 100 may be sealed or in a quasi-sealed state. The quasi-sealed state refers to a state close to sealing but not completely sealed. For example, it means a state in which the air between the inside and the outside of the decontamination object 100 is blocked at a certain level, and the concentration of peracetic acid does not extremely decrease due to leakage of peracetic acid. In the present embodiment, the decontamination object 100 is a safety cabinet. Hereinafter, the decontamination object 100 may be referred to as a safety cabinet 100. The decontamination system 10 includes a decontamination device 20 and a decontamination intensity sensor 60.

In the decontamination system 10, the decontamination device 20 is connected to the safety cabinet 100 via pipes 91 and 92. For example, at least one of microorganisms and viruses existing inside the safety cabinet 100 is decontaminated by the decontamination device 20. In the present embodiment, the decontamination device 20 is disposed outside the safety cabinet 100.

The safety cabinet 100 is box-shaped experimental equipment for suppressing biohazard. The experimenter inserts a hand into a work area WA and performs an experiment using a biological material, for example. The safety cabinet 100 includes a fan 110, high efficiency particulate air (HEPA) filters 120 and 130, and a shutter 140. During the experiment, an air flow is generated by the operation of the fan 110, clean air is discharged to the outside through the HEPA filter 120, and the clean air is supplied to the work area WA through the HEPA filter 130. The shutter 140 is configured to be openable and closable. The filter 120 may be an ultra low penetration air (ULPA) filter. The filter 120 is covered with a cover portion 100Z attached to the safety cabinet 100, for example. The cover portion 100Z is attached to the safety cabinet 100 at the start of decontamination, for example.

In the safety cabinet 100, for example, communication holes 100A, 100B, 100C, and 100D are formed. The communication holes 100A to 100D may be installed exclusively for decontamination, or for example, a drain portion, a vacuum, and a DOP sampling port provided in a general safety cabinet may be used as the communication holes 100A to 100D. Each of the communication holes 100A to 100D can be opened and closed. It is preferable that the communication holes 100A, 100B, 100C, and 100D are sealed when the safety cabinet 100 is used. It is preferable that the communication holes 100A, 100B, 100C, and 100D are sealed at the time of decontamination of the safety cabinet 100 except when connected to the decontamination device 20. In the example shown in Fig. 1, the decontamination device 20 is connected to the communication hole 100B via the pipe 92, and the decontamination device 20 is connected to the communication hole 100D via the pipe 91. That is, the decontamination device 20 is connected to the communication hole 100D in the ceiling portion of the safety cabinet 100 and the communication hole 100B in the bottom portion of the safety cabinet 100. The number of communication holes connecting the pipe 92 may be one or two or more.

During the decontamination, the shutter 140 is closed. Even when the shutter 140 is closed, the inside and the outside of the safety cabinet 100 may not be completely blocked, and in such a case, a slight gap is formed. During the decontamination, the gap is cured by a curing tape 200 in order to prevent the leakage of the decontamination gas depending on the degree of the leakage of the decontamination gas. It is preferable that each of the communication holes 100A and 100C is closed.

<2. Configuration of Decontamination Device>

The decontamination device 20 includes a storage device 30, a control device 40, and an ejection device 50. The storage device 30 and the control device 40 are connected so as to be able to communicate via a bus (not shown). The storage device 30 is, for example, a hard disk or a solid state drive. The storage device 30 stores one or a plurality of programs (hereinafter, it is referred to as an "ejection program 30P") related to the control of the operation of the ejection device 50.

The control device 40 includes a central processing unit (CPU), a random access memory (RAM), a read only memory (ROM), and the like, and executes functions to be described later. The control device 40 executes the ejection program 30P stored in the storage device 30 in response to a request from a user. When the control device 40 executes the ejection program 30P, one or a plurality of functional blocks for controlling the operation of the ejection device 50 are constructed in the decontamination device 20. The one or the plurality of functional blocks include an ejection control unit 40X.

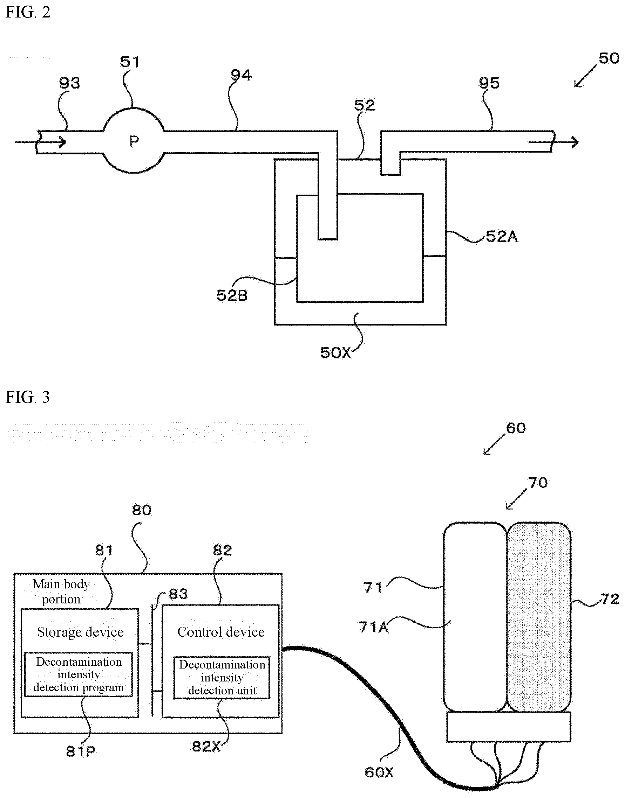

Fig. 2 is a diagram showing a schematic configuration of the ejection device 50. The ejection device 50 includes a pump 51 and a vapor generator 52. Pipes 93 and 94 are connected to the pump 51. The pipe 93 is connected to the pipe 91 (see Fig. 1). The pump 51 is configured to suck air from the pipe 93 side and supply air to the pipe 94 side.

In the present embodiment, the vapor generator 52 is configured to generate only vapor containing peracetic acid without heating a chemical solution 50X and releasing mist. The vapor generator 52 includes a container 52A, a moisture absorbing member 52B, and a chemical solution 50X. The container 52A is, for example, a cylindrical sealed container. The container 52A accommodates the moisture absorbing member 52B and the chemical solution 50X. The chemical solution 50X is a liquid chemical containing peracetic acid. More specifically, the chemical solution 50X is a peracetic acid preparation.

The structure and member of the moisture absorbing member 52B are not particularly limited as long as it is wetted by the chemical solution 50X and the chemical solution 50X can be efficiently gasified (vaporized) by ventilation. For example, the moisture absorbing member 52B may use a sheet-like member such as a woven fabric, a knitted fabric, a nonwoven fabric, or a film as it is, or may be formed by processing into a pleated shape or a corrugated shape. A porous material such as silica gel or zeolite may be contained in a woven fabric, a knitted fabric, a nonwoven fabric, a film, or the like. In the present embodiment, the moisture absorbing member 52B is made of, for example, a porous material. The moisture absorbing member 52B is immersed in the chemical solution 50X. The chemical solution 50X is impregnated into the moisture absorbing member 52B. The moisture absorbing member 52B sucks up the chemical solution 50X in the container 52A by a capillary phenomenon. In another example, the vapor generator 52 may have a mechanism for sucking up the chemical solution 50X and pouring the chemical solution 50X from the upper portion of the moisture absorbing member 52B, or may have a stirring fan for promoting vaporization of the chemical solution 50X.

The pipe 94 and the pipe 95 are connected to the container 52A. The pipe 95 is connected to the pipe 92 (see Fig. 1). The position and length of the pipes 94 and 95 connected to the container 52A in the container 52A are not particularly limited. Since it is preferable that the pipes 94 and 95 enter the chemical solution 50X and the chemical solution 50X does not foam, it is preferable that the positions and lengths of the pipes 94 and 95 in the container 52A can realize such a configuration, for example, a configuration in which the distal ends of the pipes 94 and 95 do not enter the chemical solution 50X. In addition, it is preferable that the distal end of the pipe 94 for introducing air and the distal end of the pipe 95 for discharging air are separated from each other, the flow path of air is long, and the distal ends of the pipes 94 and 95 are present at positions where the generation of the peracetic acid vapor of the moisture absorbing member 52B is promoted.

According to the decontamination device 20, since the chemical solution 50X is not heated, decomposition of peracetic acid can be suppressed and peracetic acid vapor can be efficiently generated. Further, according to the decontamination device 20, since the temperature of the chemical solution 50X containing peracetic acid is maintained at the same level as the temperature in the space where the HEPA filters 120 and 130 to be decontaminated are disposed, the possibility of dew condensation occurring in the space can be suppressed.

Air is supplied from the pump 51 via the pipe 94, and vaporization of the chemical solution 50X impregnated into the moisture absorbing member 52B is promoted. Therefore, vapor (hereinafter, referred to as "peracetic acid vapor") containing peracetic acid is generated. The peracetic acid vapor is supplied to the inside of the safety cabinet 100 via the pipe 95. The decontamination device 20 of the present embodiment is a so-called vaporizing type that ejects peracetic acid vapor, but the decontamination device 20 may be a so-called atomizing type that ejects a mist of peracetic acid.

In the conventional decontamination system, prior to decontamination, for example, a biological indicator (BI) 150 is disposed in a space above the HEPA filter 120. In addition, a BI 160 is disposed in the work area WA. After completion of the decontamination, the BIs 150 and 160 are cultured, and the decontamination effect is confirmed based on the state of killing bacteria and the like adhering to the BIs 150 and 160.

A user can use the safety cabinet 100 when it is confirmed that culture of the BIs 150 and 160 has been completed and decontamination of the safety cabinet 100 has been completed, in other words, bacteria and the like adhering to the BIs 150 and 160 are completely killed. However, since the cultivation of the BIs 150 and 160 usually requires at least about one week, the user cannot use the safety cabinet 100 for at least about one week. The decontamination system 10 of the present embodiment includes the decontamination intensity sensor 60 so that whether or not decontamination of the decontamination object 100 has been completed can be determined in a short time.

<3. Configuration of Decontamination Intensity Sensor>

Fig. 3 is a diagram showing a schematic configuration of the decontamination intensity sensor 60. The basic configuration of the decontamination intensity sensor 60 is similar to that of a conventional electrical resistance corrosion sensor, but it is preferable to detect corrosion of metal of about 0.01 $\mu$m as measurement sensitivity. Examples of the method for measuring the electric resistance include a two-terminal measurement method and a four-terminal measurement method, and a four-terminal measurement method with better measurement sensitivity is preferable.

The decontamination intensity sensor 60 of the present embodiment includes a sensor unit 70 and a main body portion 80. The sensor unit 70 and the main body portion 80 are electrically connected by a cable 60X. The sensor unit 70 is disposed at an arbitrary position in the safety cabinet 100. For example, the sensor unit 70 is disposed at an arbitrary position in the work area WA as shown in Fig. 1. The sensor unit 70 may be disposed in a space above the HEPA filter 120, in other words, on the secondary side with respect to the HEPA filter 120. A plurality of sensor units 70 may be disposed in the safety cabinet 100. The main body portion 80 is disposed outside the safety cabinet 100. The main body portion 80 may be connected to an output device that displays a ratio RA of the electric resistance value to be described later. The output device is, for example, a liquid crystal display. The cable 60X is inserted into the work area WA via, for example, a pipe 170 (see Fig. 1) disposed in a gap below the shutter 140. During the decontamination, the gap except for the pipe 170 below the shutter 140 is cured by the curing tape 200. Note that the cable 60X may be inserted into the work area WA via the communication hole 100A or the communication hole 100C, for example.

The sensor unit 70 includes a first sensor unit 71 and a second sensor unit 72. The first sensor unit 71 is made of metal corroded by peracetic acid and has a bar shape. The corrosion of the first sensor unit 71 includes a chemical reaction in which metal is changed to acetate. Since the reaction of the metal with the acetate is used, corrosion of an exposed portion 71A described later in the sensor unit 70 rapidly proceeds. Since the electric resistance value is likely to change, corrosion can be measured with high sensitivity, and progress of decontamination can be measured with high sensitivity. Since the acetate is soluble in water, the corroded portion can be removed by wiping the corroded portion when the first sensor unit 71 is corroded with, for example, a nonwoven fabric moistened with pure water. In another example, the corroded portion can be removed by rubbing the corroded portion of the first sensor unit 71 with metal polishing using alumina or the like. When the first sensor unit 71 corrodes, the sensitivity of the first sensor unit 71 decreases due to corrosion, but the first sensor unit 71 can be used with high sensitivity by removing the corroded portion.

The first sensor unit 71 includes the exposed portion 71A having the metal exposed therefrom. The second sensor unit 72 has a bar shape similar to that of the first sensor unit 71, and is made of the same metal as the metal constituting the first sensor unit 71. The substantially entire surface of the second sensor unit 72 is masked so that metal does not corrode. The metal constituting the first sensor unit 71 and the second sensor unit 72 can be freely selected as long as it is metal corroded by peracetic acid. The metal constituting the first sensor unit 71 and the second sensor unit 72 preferably includes, for example, at least one of copper and zinc. Copper and zinc have different degrees of progress of corrosion due to peracetic acid under the same conditions. Under the same conditions, corrosion of zinc due to peracetic acid is more likely to proceed than that of copper. From the viewpoint of repeatedly using the sensor unit 70, corrosion of the first sensor unit 71 and the second sensor unit 72 may proceed within a range in which a change in the ratio RA of the electric resistance value to be described later can be grasped. Therefore, the metal constituting the first sensor unit 71 and the second sensor unit 72 is preferably an alloy containing copper and zinc so that the progress of corrosion becomes a desired degree according to the decontamination intensity. When the sensor unit 70 is used in an environment with high decontamination intensity, the metal constituting the sensor unit 70 is preferably an alloy having a high ratio of copper to zinc in order to suppress the progress of corrosion. When the sensor unit 70 is used in an environment with low decontamination intensity, the metal constituting the sensor unit 70 is preferably an alloy having a high ratio of zinc to copper in order to promote the progress of corrosion. As the metal constituting the first sensor unit 71 and the second sensor unit 72, for example, brass (C2680) can be suitably used.

The sizes of the first sensor unit 71 and the second sensor unit 72 can be freely selected. In the present embodiment, the length of the first sensor unit 71 and the second sensor unit 72 is 90 mm, and the thickness is 50 $\mu$m.

The main body portion 80 includes a storage device 81 and a control device 82. The storage device 81 and the control device 82 are connected so as to be able to communicate via a bus (not shown). The storage device 81 is, for example, a hard disk or a solid state drive. The storage device 81 stores one or a plurality of programs (hereinafter, it is referred to as a "decontamination intensity detection program 81P") related to the detection of the decontamination intensity.

The control device 82 includes a CPU, a RAM, a ROM, and the like, and executes functions to be described later. The control device 82 executes a decontamination intensity detection program 81P stored in the storage device 81 in response to a request from the user. When the control device 82 executes the decontamination intensity detection program 81P, one or a plurality of functional blocks for detecting the decontamination intensity are constructed in the main body portion 80. The one or the plurality of functional blocks include a decontamination intensity detection unit 82X.

The inventor(s) of the present application have found that when the decontamination object 100 is decontaminated with peracetic acid, corrosion of the metal constituting the first sensor unit 71 proceeds as decontamination intensity increases, and the electric resistance value increases. The degree of corrosion of the metal constituting the first sensor unit 71 can be expressed as a numerical value by the decontamination intensity sensor 60 that measures the resistance value of the metal. According to the decontamination intensity sensor 60, the corrosion of the exposed portion 71A of the first sensor unit 71 proceeds as the decontamination intensity of the decontamination object 100 increases, so that the electric resistance value of the first sensor unit 71 increases. On the other hand, since the second sensor unit 72 is masked, it does not corrode even when the decontamination intensity of the decontamination object 100 becomes high. Therefore, the electric resistance value of the second sensor unit 72 does not substantially change. Therefore, when the relationship between the electric resistance value of the first sensor unit 71 and the electric resistance value of the second sensor unit 72 satisfies the predetermined relationship in which it can be determined that decontamination has been completed, it can be determined that the decontamination has been completed. Therefore, according to the decontamination system 10, it can be determined in a short time that decontamination of the decontamination object has been completed. In the present embodiment, for example, it is determined that the decontamination has been completed when the ratio RA of the electric resistance value of the first sensor unit 71 to the electric resistance value of the second sensor unit 72 is equal to or larger than a predetermined reference ratio RAX by which it can be determined that decontamination has been completed. The decontamination intensity detection unit 82X calculates the ratio RA of the electric resistance value in real time based on the following equation (1) using an electric resistance value R1 of the first sensor unit 71 and an electric resistance value R2 of the second sensor unit 72 during the decontamination by the decontamination device 20.

$$RA = R1/R2 \cdots (1)$$

In the present embodiment, the increase in the electric resistance value R1 with the progress of corrosion of the exposed portion 71A is minute. The control device 82 of the present embodiment can measure a change in electric resistance value of 0.01%.

<4. Method for Calculating Reference Ratio RAX>

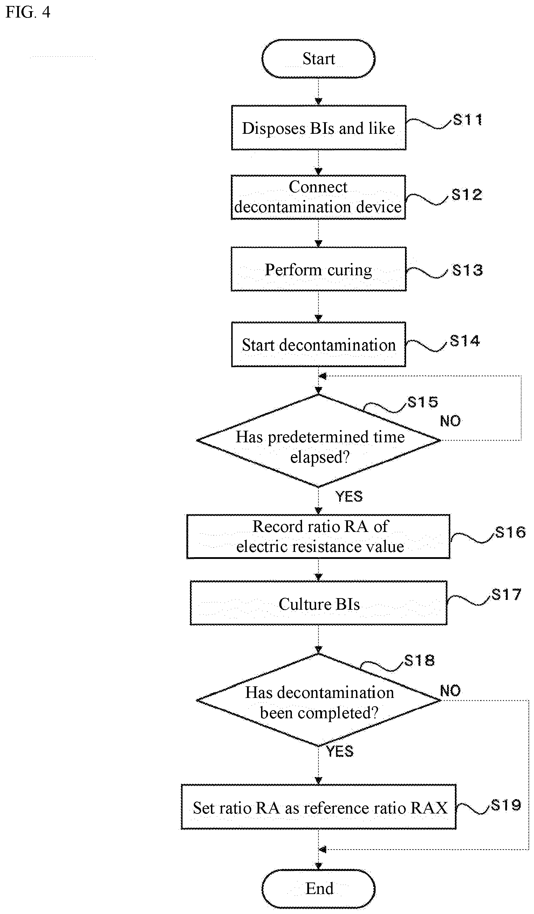

Fig. 4 is a flowchart showing an example of a procedure of a method for calculating the reference ratio RAX. Each step shown in this flowchart is performed by an operator.

In step S11, the operator disposes the BIs 150 and 160 and the sensor unit 70 of the decontamination intensity sensor 60 in the safety cabinet 100. In step S12, the operator connects the decontamination device 20 to the safety cabinet 100 using the pipes 91 and 92. In step S13, the operator provides an opening in the safety cabinet 100 as necessary, or cures the gap with the curing tape 200. In step S14, the operator drives the decontamination device 20 to start decontamination of the inside of the safety cabinet 100.

In step S15, the operator determines whether or not a predetermined time TX has elapsed. When the determination in step S15 is negative, that is, when the predetermined time TX has not elapsed, the operator repeatedly executes the determination in step S15. When the determination in step S15 is affirmative, that is, when the predetermined time TX has elapsed, the operator records the ratio RA of the electric resistance value calculated by the main body portion 80 in step S16, and stops the driving of the decontamination device 20.

In step S17, the operator causes the BIs 150 and 160 to be cultured in the culture solution. In step S18, based on the color of the culture solution, the operator determines whether or not bacteria and the like adhering to the BIs 150 and 160 have been completely killed, that is, whether or not decontamination of the safety cabinet 100 has been completed. When the determination in step S18 is affirmative, that is, when the decontamination of the safety cabinet 100 has been completed, the operator sets the ratio RA recorded in step S16 as the reference ratio RAX by which it can be determined that the decontamination has been completed. When the determination in step S18 is negative, that is, when decontamination of the decontamination object 100 is not completed, the operator terminates the present calculation method, changes the predetermined time TX, and executes the present calculation method again. In a typical example, in the present calculation method executed again, the operator determines whether or not a predetermined time TY longer than the predetermined time TX has elapsed in step S15.

<5. Decontamination Procedure>

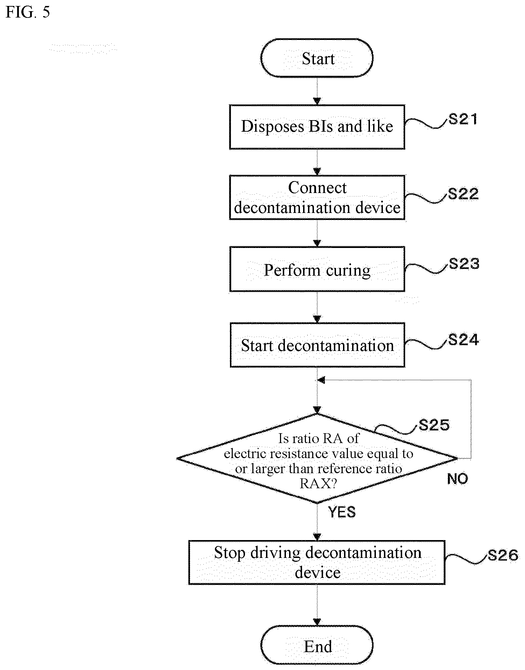

Fig. 5 is a flowchart showing an example of a decontamination procedure in the safety cabinet 100. Each step shown in this flowchart is performed by an operator. Note that steps S21 to S24 shown in Fig. 5 are the same procedures as steps S11 to S14 shown in Fig. 4, and thus the description thereof will be omitted.

In step S25, the operator determines whether the ratio RA of the electric resistance value is equal to or larger than the reference ratio RAX by which it can be determined that the predetermined decontamination has been completed. When the determination in step S25 is negative, that is, when the ratio RA of the electric resistance value is less than the reference ratio RAX, the operator repeatedly executes the determination in step S25. When the determination in step S25 is affirmative, that is, when the ratio RA of the electric resistance value is equal to or larger than the reference ratio RAX, the operator stops driving the decontamination device 20 in step S26.

<6. Operation and Effect of Decontamination System>

In the decontamination system 10, it can be determined that the decontamination has been completed when the ratio RA of the electric resistance value of the first sensor unit 71 to the electric resistance value of the second sensor unit 72 is equal to or larger than the reference ratio RAX. Therefore, it can be determined in a short time that decontamination of the decontamination object 100 has been completed. In addition, in order to complete decontamination, it is possible to suppress application of an excessive load to the equipment present in the decontamination object 100 by ejecting peracetic acid at a higher concentration than necessary and performing decontamination for a long time. Even when the ejection flow rate of the peracetic acid vapor by the ejection device 50, the concentration of the peracetic acid vapor, and the predetermined time TX are the same, whether or not the decontamination has been completed depends on various factors such as the temperature and humidity of the work area WA. Therefore, by repeatedly performing the method for calculating the reference ratio RAX to calculate the reference ratio RAX, it is possible to accurately determine whether or not the decontamination of the decontamination object 100 has been completed even when the temperature, humidity, and the like of the work area WA change.

<7. Modifications>

The above embodiment is examples of modes that can be taken by the decontamination intensity sensor, the decontamination system, the decontamination intensity detection method, and the decontamination intensity detection program according to the present invention, and is not intended to limit the modes. The decontamination intensity sensor, the decontamination system, the decontamination intensity detection method, and the decontamination intensity detection program according to the present invention can take modes different from the modes exemplified in the embodiments. An example thereof is a mode in which a part of the configuration of the embodiment is replaced, changed, or omitted, or a mode in which a new configuration is added to the embodiment. Some examples of modifications of the embodiment will be described below. Note that the following modifications can be combined with each other within a range not technically contradictory.

<7-1. First Modification>

In the above embodiment, the second sensor unit 72 can be omitted from the decontamination intensity sensor 60. In this modification, for example, the electric resistance value of the exposed portion 71A before the start of the decontamination is used instead of the electric resistance value of the second sensor unit 72, so that it can be determined that the decontamination has been completed when the relationship between the electric resistance value of the exposed portion 71A before the start of the decontamination and the electric resistance value of the exposed portion 71A after the start of the decontamination satisfies a predetermined condition under which it can be determined that the decontamination has been completed.

<7-2. Second Modification>

In the above embodiment, it may be determined that the decontamination has been completed when a thickness HA of the corroded portion of the exposed portion 71A is equal to or larger than a predetermined thickness HAX by which it can be determined that the decontamination has been completed.

<7-3. Third Modification>

In the sensor unit 70, the electric resistance value of the first sensor unit 71 may not match the electric resistance value of the second sensor unit 72 in an unused state or before the start of decontamination. In other words, the sensor unit 70 may not have the ratio RA of "1" in an unused state or before the start of decontamination. Therefore, in the above embodiment, it is preferable to correct the ratio RA using a correction coefficient K as in the following equation (2). The correction coefficient K is a reciprocal of the ratio RAS of the electric resistance value of the first sensor unit 71 to the electric resistance value of the second sensor unit 72 in an unused state or before the start of decontamination. For example, when the electric resistance value of the first sensor unit 71 in an unused state or before the start of decontamination is 1.1000 and the electric resistance value of the second sensor unit 72 is 1.0000, the ratio RAS is 1.1000, and thus the correction coefficient K is 0.9091.

$$RA = K \cdot (R1/R2) \cdots (2)$$

<7-4. Fourth Modification>

In the above embodiment, for example, the decontamination intensity detection unit 82X may determine that the decontamination has been completed when the ratio RB of the electric resistance value of the second sensor unit 72 to the electric resistance value of the first sensor unit 71 is equal to or less than a predetermined reference ratio RBX by which it can be determined that the decontamination has been completed. The decontamination intensity detection unit 82X calculates the ratio RB of the electric resistance value in real time based on the following equation (3) using an electric resistance value R1 of the first sensor unit 71 and an electric resistance value R2 of the second sensor unit 72 during the decontamination by the decontamination device 20. The reference ratio RBX can be calculated based on, for example, the same concept as the flowchart for calculating the reference ratio RAX shown in Fig. 4.

$$RB = R2/R1 \cdots (3)$$

### <7-5. Fifth Modification>

In the above embodiment, the decontamination intensity detection unit 82X may determine whether or not decontamination has been completed based on a difference $\Delta RA$ between the ratio RA and the ratio RAS of the electric resistance value of the first sensor unit 71 to the electric resistance value of the second sensor unit 72 before the start of decontamination as expressed by the following equation (4). In this modification, the decontamination intensity detection unit 82X determines that decontamination has been completed when the difference $\Delta RA$ is equal to or larger than a predetermined reference difference $\Delta RX$ by which it can be determined that the decontamination has been completed.

$$\Delta RA = RA\text{-}RAS \cdots (4)$$

### <7-6. Sixth Modification>

In the above embodiment, the decontamination intensity detection unit 82X may determine whether or not decontamination has been completed based on a difference $\Delta RB$ between a ratio RBS of the electric resistance value of the second sensor unit 72 to the electric resistance value of the first sensor unit 71 before the start of decontamination and a ratio RB of the fourth modification as expressed in the following equation (5). In this modification, the decontamination intensity detection unit 82X determines that the decontamination has been completed when the difference $\Delta RB$ is equal to or larger than a predetermined reference difference $\Delta RY$ by which it can be determined that the decontamination has been completed.

$$\Delta RB = RBS\text{-}RB \cdots (5)$$

### <7-7. Seventh Modification>

In the above embodiment, the main body portion 80 of the decontamination intensity sensor 60 can also be mounted on the decontamination device 20. In addition, the main body portion 80 may be omitted from the decontamination intensity sensor 60, and the decontamination intensity detection program 81P can also be stored in the storage device 30 of the decontamination device 20. In this modification, the control device 40 of the decontamination device 20 executes the decontamination intensity detection program 81P, so that the decontamination intensity detection unit 82X is constructed in the decontamination device 20. Further, in this modification, the decontamination intensity detection unit 82X may execute the determination processing in step S25 of the decontamination procedure shown in Fig. 5, and the ejection control unit 40X may stop the drive of the ejection device 50 when the determination in step S25 is affirmative.

### <7-8. Eighth Modification>

In the above embodiment, the decontamination intensity detection unit 82X may determine whether or not decontamination has been completed based on a difference $\Delta RC$ between the electric resistance value of the first sensor unit 71 and the electric resistance value of the second sensor unit 72. In this modification, the decontamination intensity detection unit 82X determines that decontamination has been completed when the difference $\Delta RC$ is equal to or larger than a predetermined reference difference $\Delta RZ$ by which it can be determined that the decontamination has been completed.

### <7-9. Ninth Modification>

In the above embodiment, the particle removing filters disposed in the safety cabinet 100 are the HEPA filters 120 and 130, but the particle removing filter disposed in the safety cabinet 100 is not limited thereto. The filter for removing particles to be decontaminated may be, for example, a medium-performance filter or an ULPA filter.

### <7-10. Tenth Modification>

In the above embodiment, in the decontamination device 20, the air flow is in the order of the safety cabinet 100, the pump 51, the vapor generator 52, and the safety cabinet 100, but the order of the air flow is not limited thereto. For example, the air may flow through the safety cabinet 100, the vapor generator 52, the pump 51, and the safety cabinet 100 in this order.

### <8. Tests>

In order to confirm the effect of the present invention, the inventor(s) of the present application conducted the following first test and second test using a decontamination system for testing. In addition, the inventor(s) of the present application conducted a third test for confirming metal that can be suitably used for the sensor unit 70. In the following description, for convenience of description, the same elements as those of the embodiment among the elements constituting the decontamination system for testing will be denoted by the same reference numerals as those of the embodiment.

<8-1. First Test>

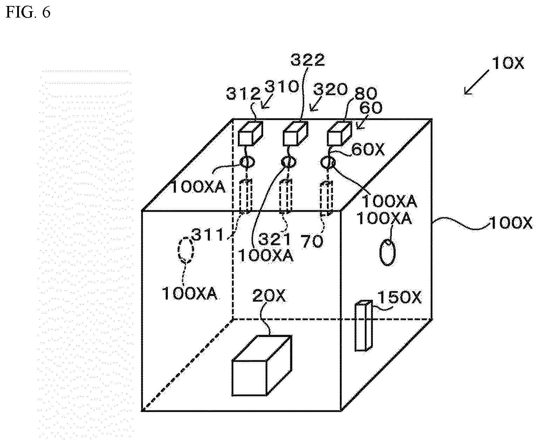

Fig. 6 is a diagram showing a schematic configuration of a decontamination system 10X used in the first test. The decontamination system 10X includes a decontamination object 100X, a decontamination device 20X, a BI 150X, a thermohygrometer 310, a peracetic acid densitometer 320, and a decontamination intensity sensor 60.

The decontamination object 100X is an acrylic box. The size of the acrylic box is 78 cm wide by 57 cm deep by 78 cm high. The acrylic box has a door (not shown) that can be opened and closed on the entire surface. The acrylic box has openings 100XA on the left and right and on the top surface. The diameter of the opening 100XA is 30 mm. From the opening 100XA on the top surface, a sensor unit 311 of the thermohygrometer 310, a sensor unit 321 of the peracetic acid densitometer 320, and a sensor unit 70 of the decontamination intensity sensor 60 are inserted into the space in the acrylic box. The sensor unit 311 of the thermohygrometer 310 is an LR9502 manufactured by HIOKI E.E. CORPORATION. The main body portion 312 of the thermohygrometer 310 is an LR5001 manufactured by HIOKI E.E. CORPORATION. The sensor unit 321 of the peracetic acid densitometer 320 is a peracetic acid gas sensor module 00-1705 manufactured by Accurate Technologies Inc.. A main body portion 322 of the peracetic acid densitometer 320 is an F12 series gas detector manufactured by Accurate Technologies Inc..

The BI 150X is disposed in a space in the acrylic box. The BI 150X is HMV-091 with a bacteria count of $10^6$ manufactured by MesaLab. The culture solution of the BI 150X is PM/100 manufactured by MesaLab.

The decontamination device 20X is disposed in a space in the acryl box. The decontamination device 20X includes a container that accommodates a peracetic acid chemical, acetic acid, or hydrogen peroxide, and a fan attached to the container. The fan blows air to these liquid surfaces so that the peracetic acid chemical, acetic acid, or hydrogen peroxide vaporizes.

In Test Examples 1 to 7, a peracetic acid chemical is stored in a container of the decontamination device 20X. The peracetic acid chemical is MINNCARE manufactured by MEDIVATORS Inc.. MINNCARE has a hydrogen peroxide content of 22.0%, an acetic acid content of 4.5%, and a peracetic acid content of 9.0%. MINNCARE is diluted with pure water, and 500 ml of the diluted solution is stored in a container of the decontamination device 20X.

In Test Example 8, 500 ml of a diluted solution obtained by diluting acetic acid to 0.45% with pure water is stored in the container of the decontamination device 20X. In Test Example 9, 500 ml of a diluted solution obtained by diluting hydrogen peroxide to 1.1% with pure water is stored in the container of the decontamination device 20X.

In the first test, the ratio RA of the electric resistance value of the sensor unit 70 was measured by the decontamination intensity sensor 60 during the driving of the decontamination device 20X. In this test, the decontamination device 20X was driven for a predetermined time, and after the drive of the decontamination device 20X was stopped, the BI 150X was taken out from the left and right openings 100XA of the acrylic box, and the BI 150X was cultured in the culture solution.

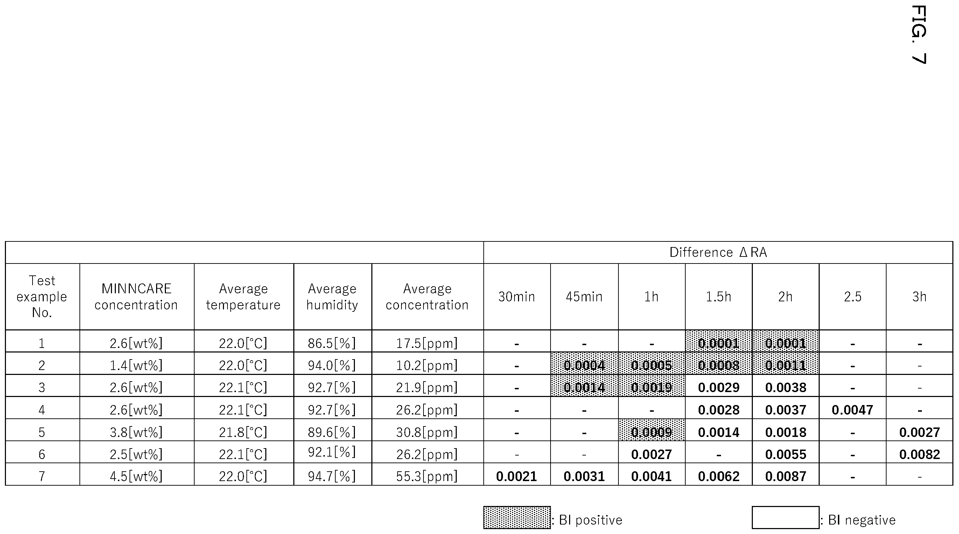

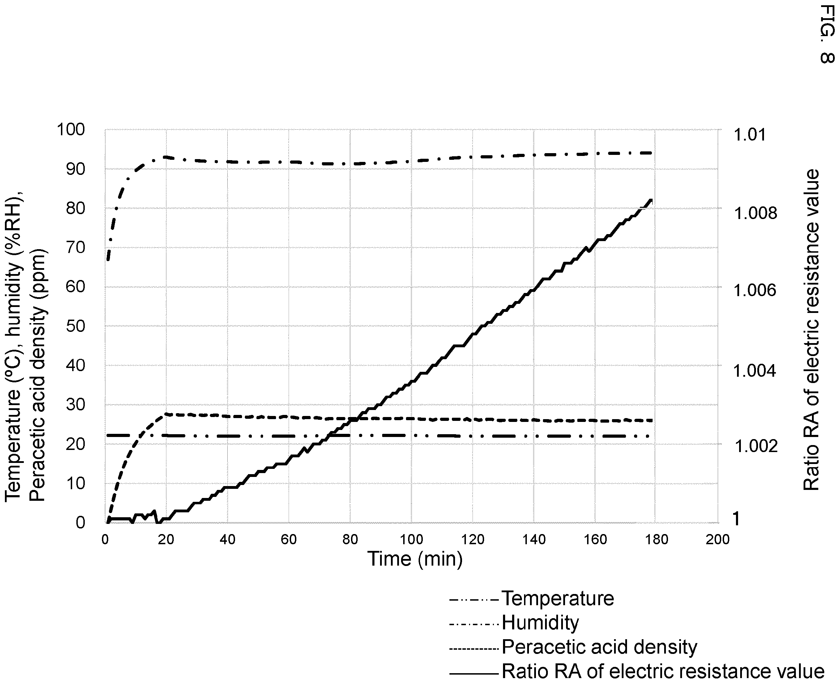

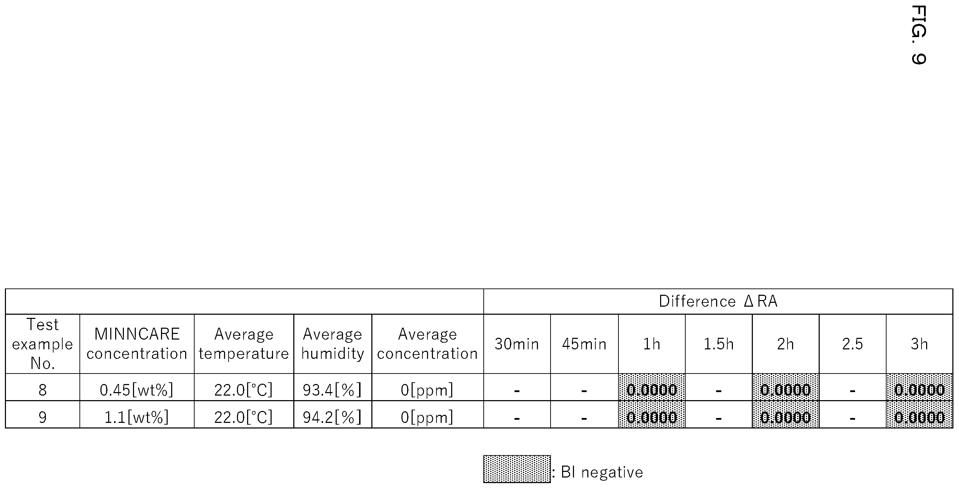

Fig. 7 is a table showing test results of Test Examples 1 to 7. Fig. 8 is a graph showing test results of Test Example 6. Fig. 9 is a table showing test results of Test Examples 8 and 9. In this test, it was confirmed that the BI 150X was killed and the decontamination was completed when the difference ΔRA in electric resistance value shown in the fifth modification was 0.0021 or more. In addition, from Test Examples 2, 3, and 7, it was confirmed that the difference ΔRA in electric resistance value was different when the concentration of peracetic acid was different even when the humidity was comparable. From Test Examples 1 and 3, it was confirmed that the difference ΔRA in electric resistance value was different when the humidity was different even when the concentration of peracetic acid was about the same. Whether or not the decontamination of the decontamination object 100X has been completed depends on various factors such as temperature, humidity, and decontamination time, and it was confirmed that whether or not the decontamination of the decontamination object 100X has been completed can be accurately determined in a short time by using the ratio RA of the electric resistance value detected by the decontamination intensity sensor 60. From Test Examples 8 and 9, it was confirmed that the BI 150X was not killed by acetic acid or hydrogen peroxide, and an exposed portion 71X of the first sensor unit 71 was not corroded.

<8-2. Second Test>

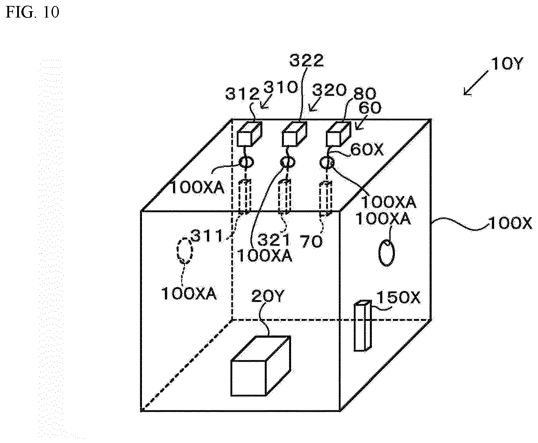

Fig. 10 is a diagram showing a schematic configuration of a decontamination system 10Y used in the second test. The decontamination system 10Y differs from the decontamination system 10X used in the first test in the configuration of a decontamination device 20Y, and the other configurations are the same as those of the decontamination system 10X. The decontamination device 20Y is an ultrasonic mist generator. The ultrasonic mist generator is FOGACT manufactured by Nitta Corporation. The test method of the second test is the same as that of the first test.

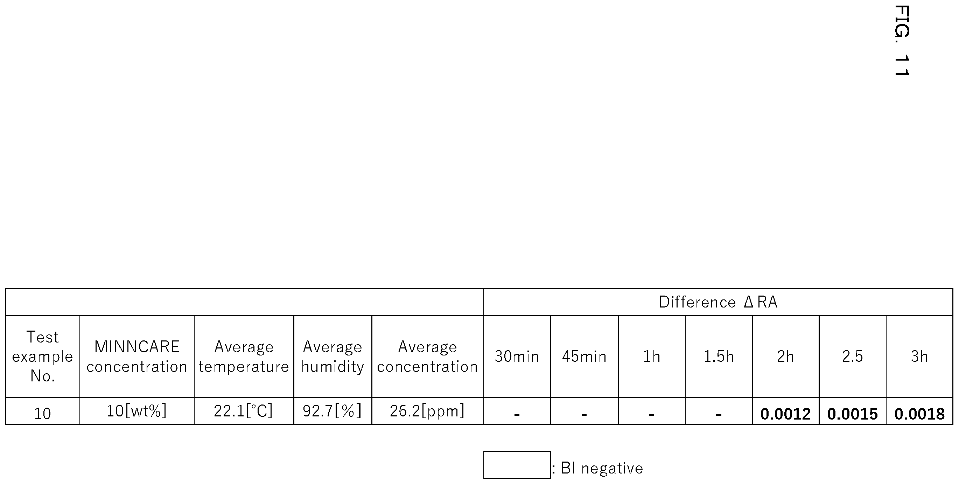

Fig. 11 is a table showing test results of the second test. In the second test, from Test Example 9, it was confirmed that the BI 150X was killed and the decontamination was completed when the difference ΔRA in electric resistance value shown in the fifth modification was 0.0012 or more. It was confirmed that even if the decontamination device 20Y is an ultrasonic mist generator, whether or not decontamination of the decontamination object 100X was completed can be accurately determined in a short time by measuring the ratio RA of the electric resistance value.

In addition, from the results of the first test and the second test, it was confirmed that the degree of progress of metal corrosion caused by peracetic acid is related to the degree of killing or damaging microorganisms and viruses by decontamination. As a result, the degree to which the microorganisms and viruses are killed or damaged by decontamination can be expressed as a numerical value indicated by the decontamination intensity sensor 60, in other words, the decontamination intensity. Furthermore, the timing at which decontamination should be completed can be grasped by the decontamination intensity indicated by the decontamination intensity sensor 60 when the microorganisms and viruses are killed.

<8-3. Third Test>

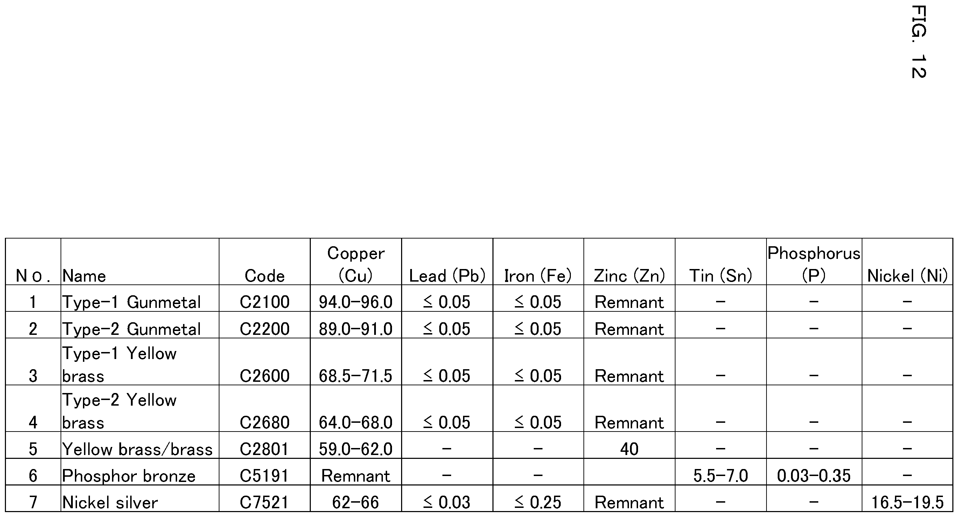

Fig. 12 is a table showing specifications of metal constituting the sensor unit 70 used in the third test. In the third test, the metals of the samples No. 1 to No. 7 shown in Fig. 12 were accommodated in the decontamination object 100X of the decontamination system 10X shown in Fig. 6, and peracetic acid was ejected by the decontamination device 20X. In the third test, the opening 100XA of the decontamination object 100X is sealed. As the BI 150X, in addition to HMV-091 with a bacteria count of $10^6$ manufactured by MesaLab in the first test, LCD-404 with a bacteria count of $10^4$ manufactured by MesaLab was used. The culture solutions of the two BIs 150X are PM/100 manufactured by MesaLab. The metals of the samples No. 1 to No. 7 are samples defined by JIS H 3100 "Plates and Strips of Copper and Copper Alloy".

In the third test, the BIs 150X were taken out at 2 hours, 3 hours, and 4 hours elapsed after the start of decontamination, and it was confirmed whether or not the BIs 150X were killed. In the third test, it was confirmed that the two BIs 150X had been killed, in other words, were negative, at all 2 hours, 3 hours, and 4 hours elapsed.

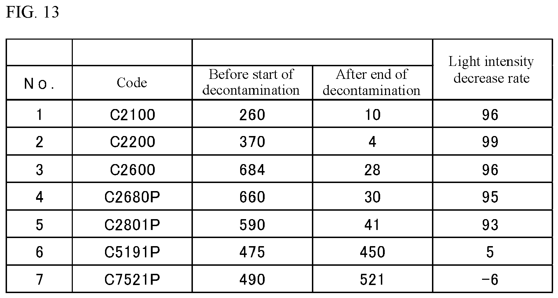

In the third test, gloss of the metal before the start of decontamination and after the completion of decontamination was measured using a gloss meter (gloss checker IG-410 manufactured by HORIBA, Ltd.) in order to confirm the degree of progress of corrosion of the metals of the samples No. 1 to No. 7. Fig. 13 shows the results. The numerical value in the column of metal in Fig. 13 indicates the content ratio of metal of each sample.

As shown in Fig. 13, since the glossiness of the metals of the samples No. 1 to No. 5 significantly decreases after completion of decontamination, it can be grasped that corrosion is suitably progressing. The metal of the sample No. 6 has almost no decrease in glossiness. From this, it was confirmed that when tin or phosphorus is contained in the metal constituting the sensor unit 70, corrosion does not substantially proceed depending on peracetic acid. The metal of the sample No. 7 has no decrease in glossiness. From this, it was confirmed that when nickel is contained in the metal constituting the sensor unit 70, corrosion does not substantially proceed depending on peracetic acid.

DESCRIPTION OF REFERENCE SIGNS

10: Decontamination system
20: Decontamination device
60: Decontamination intensity sensor
71: First sensor unit
71A: Exposed portion
72: Second sensor unit
81P: Decontamination intensity detection program

Claims

1. A decontamination intensity sensor that detects a degree of decontamination of at least one of microorganisms and viruses decontaminated with peracetic acid,
the decontamination intensity sensor comprising an exposed portion being made of metal corroded by the peracetic acid, the metal being exposed from the exposed portion.

2. The decontamination intensity sensor according to claim 1, wherein
the corrosion includes a chemical reaction in which the metal is changed to acetate.

3. The decontamination intensity sensor according to claim 1 or 2, wherein
the metal contains at least one of copper and zinc.

4. The decontamination intensity sensor according to claim 3, wherein
the metal is an alloy containing the copper and the zinc.

5. The decontamination intensity sensor according to claim 1 or 2, further comprising:

a first sensor unit including the exposed portion; and
a second sensor unit made of the metal and masked so as not to corrode the metal.

6. A decontamination system comprising:

the decontamination intensity sensor according to claim 1 or 2; and
a decontamination device that ejects the peracetic acid to a decontamination object in which at least one of the microorganisms and the viruses is present.

7.  The decontamination system according to claim 6, wherein
    the decontamination device is configured to stop ejection of the peracetic acid when a progress of corrosion of the exposed portion satisfies a predetermined condition under which it can be determined that decontamination has been completed.

8.  A decontamination intensity detection method for detecting a degree of decontamination of at least one of microorganisms and viruses decontaminated with peracetic acid using a decontamination intensity sensor,

    the decontamination intensity sensor including an exposed portion being made of metal corroded by the peracetic acid, the metal being exposed from the exposed portion,
    the decontamination intensity detection method comprising a step of determining whether or not a progress of corrosion of the exposed portion satisfies a predetermined condition under which it can be determined that decontamination has been completed.

9.  A decontamination intensity detection program that detects a degree of decontamination of at least one of microorganisms and viruses decontaminated with peracetic acid using a decontamination intensity sensor,

    wherein the decontamination intensity sensor includes an exposed portion being made of metal corroded by the peracetic acid, the metal being exposed from the exposed portion, and
    wherein the decontamination intensity detection program causes a computer to execute a step of determining whether or not a progress of corrosion of the exposed portion satisfies a predetermined condition under which it can be determined that decontamination has been completed.

FIG.1

FIG. 2

FIG. 3

FIG. 4

Start

Disposes BIs and like — S11

Connect
decontamination device — S12

Perform curing — S13

Start decontamination — S14

S15
Has predetermined time
elapsed? — NO

YES

Record ratio RA of
electric resistance value — S16

Culture BIs — S17

S18
Has decontamination
been completed? — NO

YES

Set ratio RA as reference ratio RAX — S19

End

FIG. 5

```
                    ┌──────────────┐
                    │    Start     │
                    └──────┬───────┘
                           │
                    ┌──────▼───────────┐
                    │ Disposes BIs and │   S21
                    │      like        │
                    └──────┬───────────┘
                           │
                    ┌──────▼───────────┐
                    │     Connect      │   S22
                    │ decontamination  │
                    │     device       │
                    └──────┬───────────┘
                           │
                    ┌──────▼───────────┐
                    │  Perform curing  │   S23
                    └──────┬───────────┘
                           │
                    ┌──────▼───────────┐
                    │      Start       │   S24
                    │ decontamination  │
                    └──────┬───────────┘
                           │
                    ┌──────▼───────────┐
                    │   Is ratio RA of │   S25
              NO    │ electric resistance value equal to │
            ◄───────┤ or larger than reference ratio     │
                    │        RAX?      │
                    └──────┬───────────┘
                          YES
                    ┌──────▼───────────┐
                    │ Stop driving     │   S26
                    │ decontamination  │
                    │     device       │
                    └──────┬───────────┘
                           │
                    ┌──────▼───────┐
                    │     End      │
                    └──────────────┘
```

FIG. 6

FIG. 7

EP 4 454 672 A1

| Test example No. | MINNCARE concentration | Average temperature | Average humidity | Average concentration | Difference ΔRA | | | | | | |
|---|---|---|---|---|---|---|---|---|---|---|---|
| | | | | | 30min | 45min | 1h | 1.5h | 2h | 2.5 | 3h |
| 1 | 2.6[wt%] | 22.0[°C] | 86.5[%] | 17.5[ppm] | - | - | - | 0.0001 | 0.0001 | - | - |
| 2 | 1.4[wt%] | 22.0[°C] | 94.0[%] | 10.2[ppm] | - | 0.0004 | 0.0005 | 0.0008 | 0.0011 | - | - |
| 3 | 2.6[wt%] | 22.1[°C] | 92.7[%] | 21.9[ppm] | - | 0.0014 | 0.0019 | 0.0029 | 0.0038 | - | - |
| 4 | 2.6[wt%] | 22.1[°C] | 92.7[%] | 26.2[ppm] | - | - | - | 0.0028 | 0.0037 | 0.0047 | - |
| 5 | 3.8[wt%] | 21.8[°C] | 89.6[%] | 30.8[ppm] | - | - | 0.0009 | 0.0014 | 0.0018 | - | 0.0027 |
| 6 | 2.5[wt%] | 22.1[°C] | 92.1[%] | 26.2[ppm] | - | - | 0.0027 | - | 0.0055 | - | 0.0082 |
| 7 | 4.5[wt%] | 22.0[°C] | 94.7[%] | 55.3[ppm] | 0.0021 | 0.0031 | 0.0041 | 0.0062 | 0.0087 | - | - |

▒ : BI positive          □ : BI negative

FIG. 8

FIG. 9

EP 4 454 672 A1

| Test example No. | MINNCARE concentration | Average temperature | Average humidity | Average concentration | Difference ΔRA | | | | | | |
|---|---|---|---|---|---|---|---|---|---|---|---|
| | | | | | 30min | 45min | 1h | 1.5h | 2h | 2.5 | 3h |
| 8 | 0.45[wt%] | 22.0[°C] | 93.4[%] | 0[ppm] | - | - | 0.0000 | - | 0.0000 | - | 0.0000 |
| 9 | 1.1[wt%] | 22.0[°C] | 94.2[%] | 0[ppm] | - | - | 0.0000 | - | 0.0000 | - | 0.0000 |

: BI negative

FIG. 10

FIG. 11

| Test example No. | MINNCARE concentration | Average temperature | Average humidity | Average concentration | Difference ΔRA | | | | | | |
|---|---|---|---|---|---|---|---|---|---|---|---|
| | | | | | 30min | 45min | 1h | 1.5h | 2h | 2.5 | 3h |
| 10 | 10[wt%] | 22.1[°C] | 92.7[%] | 26.2[ppm] | - | - | - | - | 0.0012 | 0.0015 | 0.0018 |

☐ : BI negative

FIG. 12

EP 4 454 672 A1

| No. | Name | Code | Copper (Cu) | Lead (Pb) | Iron (Fe) | Zinc (Zn) | Tin (Sn) | Phosphorus (P) | Nickel (Ni) |
|---|---|---|---|---|---|---|---|---|---|
| 1 | Type-1 Gunmetal | C2100 | 94.0–96.0 | ≤ 0.05 | ≤ 0.05 | Remnant | – | – | – |
| 2 | Type-2 Gunmetal | C2200 | 89.0–91.0 | ≤ 0.05 | ≤ 0.05 | Remnant | – | – | – |
| 3 | Type-1 Yellow brass | C2600 | 68.5–71.5 | ≤ 0.05 | ≤ 0.05 | Remnant | – | – | – |
| 4 | Type-2 Yellow brass | C2680 | 64.0–68.0 | ≤ 0.05 | ≤ 0.05 | Remnant | – | – | – |
| 5 | Yellow brass/brass | C2801 | 59.0–62.0 | – | – | 40 | – | – | – |
| 6 | Phosphor bronze | C5191 | Remnant | – | – | | 5.5–7.0 | 0.03–0.35 | – |
| 7 | Nickel silver | C7521 | 62–66 | ≤ 0.03 | ≤ 0.25 | Remnant | – | – | 16.5–19.5 |

FIG. 13

| N o. | Code | Before start of decontamination | After end of decontamination | Light intensity decrease rate |
|---|---|---|---|---|
| 1 | C2100 | 260 | 10 | 96 |
| 2 | C2200 | 370 | 4 | 99 |
| 3 | C2600 | 684 | 28 | 96 |
| 4 | C2680P | 660 | 30 | 95 |
| 5 | C2801P | 590 | 41 | 93 |
| 6 | C5191P | 475 | 450 | 5 |
| 7 | C7521P | 490 | 521 | −6 |

## INTERNATIONAL SEARCH REPORT

| International application No. |
| --- |
| **PCT/JP2022/034638** |

| A. | CLASSIFICATION OF SUBJECT MATTER |
| --- | --- |

*A61L 101/20*(2006.01)n; *A61L 2/28*(2006.01)i; *C12M 1/00*(2006.01)i; *C12M 1/12*(2006.01)i
FI:  A61L2/28; C12M1/00 A; C12M1/00 K; C12M1/12; A61L101:20

According to International Patent Classification (IPC) or to both national classification and IPC

| B. | FIELDS SEARCHED |
| --- | --- |

Minimum documentation searched (classification system followed by classification symbols)

A61L2/00; A61L9/00;C12M1/00;G01N17/00;G01N27/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2021-74033 A (NITTA KK) 20 May 2021 (2021-05-20)<br>   claims, paragraph [0019] | 1-3, 6 |
| A | same as above | 4-5, 7-9 |
| Y | WO 2013/042179 A1 (HITACHI, LTD.) 28 March 2013 (2013-03-28)<br>   claims, paragraph [0048] | 1-3, 6 |
| A | same as above | 4-5, 7-9 |
| A | JP 2000-515035 A (AMERICAN STERILIZER COMPANY) 14 November 2000<br>(2000-11-14)<br>   claims | 1-9 |
| A | JP 2008-504515 A (AMERICAN STERILIZER COMPANY) 14 February 2008 (2008-02-14)<br>   claims | 1-9 |
| A | JP 2003-315254 A (HITACHI, LTD.) 06 November 2003 (2003-11-06)<br>   claims | 1-9 |

| ✓ Further documents are listed in the continuation of Box C. | ✓ See patent family annex. |
| --- | --- |

| * Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |
| --- | --- |

| Date of the actual completion of the international search | Date of mailing of the international search report |
| --- | --- |
| **11 October 2022** | **08 November 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
| --- |
| **PCT/JP2022/034638** |

### C.    DOCUMENTS CONSIDERED TO BE RELEVANT

| Category* | Citation of document, with indication, where appropriate, of the relevant passages | Relevant to claim No. |
| --- | --- | --- |
| A | JP 2011-530085 A (PATEL, G.) 15 December 2011 (2011-12-15) <br> claims | 1-9 |

Form PCT/ISA/210 (second sheet) (January 2015)

## INTERNATIONAL SEARCH REPORT
### Information on patent family members

International application No.

**PCT/JP2022/034638**

| Patent document cited in search report | | | Publication date (day/month/year) | Patent family member(s) | | Publication date (day/month/year) |
|---|---|---|---|---|---|---|
| JP | 2021-74033 | A | 20 May 2021 | (Family: none) | | |
| WO | 2013/042179 | A1 | 28 March 2013 | US 2014/0190239 claims, paragraphs [0099]-[0100] | A1 | |
| | | | | CN 103733043 | A | |
| | | | | KR 10-2014-0047134 | A | |
| JP | 2000-515035 | A | 14 November 2000 | US 5882590 claims | A | |
| | | | | WO 1998/000176 | A1 | |
| | | | | EP 969881 | A1 | |
| | | | | AU 3515897 | A | |
| | | | | AR 8256 | A | |
| | | | | TW 358155 | B | |
| JP | 2008-504515 | A | 14 February 2008 | US 2005/0001634 claims | A1 | |
| | | | | US 2004/0178802 | A1 | |
| | | | | US 2004/0178799 | A1 | |
| | | | | US 2004/0178803 | A1 | |
| | | | | US 2004/0178804 | A1 | |
| | | | | US 2004/0263177 | A1 | |
| | | | | US 2005/0001630 | A1 | |
| | | | | US 2005/0017728 | A1 | |
| | | | | WO 2006/009563 | A1 | |
| | | | | WO 2004/083808 | A2 | |
| | | | | WO 2004/083809 | A2 | |
| | | | | WO 2004/083812 | A2 | |
| | | | | WO 2004/083813 | A2 | |
| | | | | WO 2006/022750 | A1 | |
| | | | | WO 2006/022751 | A1 | |
| | | | | WO 2006/025836 | A1 | |
| | | | | EP 1756596 | A1 | |
| | | | | CA 2570974 | A | |
| | | | | TW 200600799 | A | |
| | | | | KR 10-2007-0032996 | A | |
| | | | | CN 101036059 | A | |
| | | | | AU 2004321733 | A | |
| | | | | MX PA06014867 | A | |
| | | | | AT 511090 | T | |
| | | | | TW 200512449 | A | |
| | | | | AU 2004221488 | A | |
| | | | | TW 200519380 | A | |
| JP | 2003-315254 | A | 06 November 2003 | (Family: none) | | |
| JP | 2011-530085 | A | 15 December 2011 | US 2009/0301382 claims | A1 | |
| | | | | US 2017/0252471 | A1 | |
| | | | | US 2017/0261381 | A1 | |
| | | | | US 2018/0024011 | A1 | |
| | | | | US 2021/0322587 | A1 | |
| | | | | US 2016/0349088 | A1 | |
| | | | | US 2011/0003279 | A1 | |
| | | | | WO 2009/149243 | A1 | |

Form PCT/ISA/210 (patent family annex) (January 2015)

INTERNATIONAL SEARCH REPORT
Information on patent family members

International application No.

**PCT/JP2022/034638**

| Patent document cited in search report | Publication date (day/month/year) | Patent family member(s) | | Publication date (day/month/year) |
|---|---|---|---|---|
| | | EP 2288879 | A1 | |
| | | EP 3293493 | A1 | |
| | | AU 2009256212 | A | |
| | | CA 2726993 | A | |
| | | CN 102077060 | A | |
| | | IL 209734 | A | |
| | | KR 10-2012-0010557 | A | |
| | | RU 2010154102 | A | |
| | | AU 2016202637 | A | |
| | | ZA 201009026 | B | |
| | | AU 2017265092 | A | |
| | | BR PI0913415 | B | |
| | | AU 2019279940 | A | |
| | | CA 3115327 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6067384 B